# EUROPEAN PATENT APPLICATION

(11) **EP 2 450 072 A1**
(43) Date of publication of application: **09.05.2012**
(21) Application number: 10189781.7
(22) Date of filing: 03.11.2010
(51) Int. Cl.: A61M 5/24

(54) **Medicated module with floating piston**

(71) Applicant: Sanofi-Aventis Deutschland GmbH, 65929 Frankfurt (DE)
(72) Inventor: Davies, James, Warwickshire, CV32 7XB (GB); Wimpenny, Steven, CV 31 3QH, Warwickshire (GB)

(57) **Abstract**

A system for sequentially dispensing a non-user settable dose of one medicament followed by a user-settable dose of a primary medicament. The system may include a medicated module (202) attachable to a drug delivery device, the drug delivery device having a drug reservoir holding a first medicament. The medicated module includes a first needle (206), second needle (208), and a retention feature (212) fixed within a body of the medicated module. The module includes a recess (214) within the body of the medicated module and a piston (210) is slidably engaged with the recess. The module further includes a second medicament located in a first cavity (224) in the recess, where the first cavity is defined by a distal end of the piston and a distal end of the recess. The module also includes a diverter feature (228) that allows sequential delivery of the second medicament followed by the primary medicament.

## Description

### Field of the Present Patent Application

This present patent application relates to medical devices and methods of delivering at least two drug agents from separate reservoirs using devices having only a single dose setting mechanism and a single dispense interface. A single delivery procedure initiated by the user causes a non-user settable dose of a second drug agent and a variable set dose of a first drug agent to be delivered to the patient. The drug agents may be available in two or more reservoirs, containers or packages, each containing independent (single drug compound) or pre-mixed (co-formulated multiple drug compounds) drug agents. Specifically, this application concerns methods and systems for sequentially dispensing a non-user settable dose of one medicament followed by a user-settable dose of a primary medicament. The disclosed methods and systems use a piston and a diverter feature that in combination facilitate sequential dispense of the doses of medicament.

### Background

Certain disease states require treatment using one or more different medicaments. Some drug compounds need to be delivered in a specific relationship with each other in order to deliver the optimum therapeutic dose. The presently proposed devices and methods are of particular benefit where combination therapy is desirable, but not possible in a single formulation for reasons such as, but not limited to, stability, compromised therapeutic performance and toxicology.

For example, in some cases it might be beneficial to treat a diabetic with a long acting insulin and with a glucagon-like peptide-1 (GLP-1), which is derived from the transcription product of the proglucagon gene. GLP-1 is found in the body and is secreted by the intestinal L cell as a gut hormone. GLP-1 possesses several physiological properties that make it (and its analogs) a subject of intensive investigation as a potential treatment of diabetes mellitus.

There are a number of potential problems when delivering two active medicaments or "agents" simultaneously. The two active agents may interact with each other during the long-term, shelf life storage of the formulation. Therefore, it is advantageous to store the active components separately and combine them at the point of delivery, e.g. injection, needle-less injection, pumps, or inhalation. However, the process for combining the two agents needs to be simple and convenient for the user to perform reliably, repeatedly and safely.

A further problem is that the quantities and/or proportions of each active agent making up the combination therapy may need to be varied for each user or at different stages of their therapy. For example one or more active agents may require a titration period to gradually introduce a patient up to a "maintenance" dose. A further example would be if one active agent requires a non-adjustable fixed dose while the other is varied in response to a patient's symptoms or physical condition. This problem means that pre-mixed formulations of multiple active agents may not be suitable as these pre-mixed formulations would have a fixed ratio of the active components, which could not be varied by the healthcare professional or user.

Additional problems arise where a multi-drug compound therapy is required, because many users cannot cope with having to use more that one drug delivery system or make the necessary accurate calculation of the required dose combination. This is especially true for users with dexterity or computational difficulties. Further, for some drug combinations for which this delivery of two medicaments in a single injection step is desirable, it may be additionally desirable for the two medicaments to be delivered sequentially (i.e., one after the other, with minimal or no opportunity for mixing. Avoidance of mixing of the 2 drug formulations might have several advantages. For example, it is known that the pharmacokinetics of certain drugs is critically dependent on their concentration. By delivering 2 drugs sequentially with no mixing the optimal concentration of each drug for optimal pharmacokinetics can be maintained. In addition, certain drugs have to be formulated in particular solvent environments (e.g., a specific pH range) to remain in solution. By delivering 2 drugs sequentially with no mixing the optimal pH range and therefore solubility can be maintained during delivery.

Accordingly, there exists a need to provide devices and methods for the sequential delivery of two or more medicaments in a single injection or delivery step that is simple for the user to perform. The presently proposed devices and methods overcome the above-mentioned problems by providing separate storage containers for two or more active drug agents. The drug agents are then delivered sequentially to the patient during a single delivery procedure. Beneficially, delivering the drugs sequentially may avoid or limit mixing of the drug agents. A user sets a dose of one medicament (i.e., a user settable dose). The dose of the second medicament is independently controlled and therefore is not influenced by an amount of the user settable dose (i.e., non-user settable). The drug agents are then delivered to the patient during a single delivery procedure.

The proposed devices and methods also give the opportunity for varying the quantity of one or both medicaments. For example, one fluid quantity can be varied by changing the properties of the injection device (e.g., dialing a user variable dose or changing the device's "fixed" dose). The second fluid quantity can be changed by manufacturing a variety of secondary drug containing packages with each variant containing a different volume and/or concentration of the second active agent. The user or healthcare professional would then select the most appropriate secondary package or series or combination of series of different packages for a particular treatment regime. The proposed medicated module forms a self-contained reservoir in which non-user-settable dose of a medicament may be stored.

These and other advantages will become evident from the following more detailed description of the invention.

### SUMMARY

The presently proposed devices and methods allow for complex combinations of multiple drug compounds within a single drug delivery system. Further, the presently proposed devices and methods allow the user to sequentially dispense at least two drug agents through one single dose setting mechanism and a single dispense interface. This single dose setter controls the mechanism of the device such that a predefined combination of the individual drug compounds is delivered when a single dose of one of the medicaments is set and dispensed through the single dispense interface.

By defining the therapeutic relationship between the individual drug compounds, the proposed delivery device and delivery methods help ensure that a patient/user receives the optimum therapeutic combination dose from a multi-drug compound device without the inherent risks associated with multiple inputs where the user has to calculate and set the correct dose combination every time they use the device. The medicaments can be fluids, defined herein as liquids or gases or powders that are capable of flowing and that change shape at a steady rate when acted upon by a force tending to change its shape.

Applicants' proposed concept is of particular benefit to users with dexterity or computational difficulties as the single input and associated predefined therapeutic profile removes the need for them to calculate their prescribed dose every time they use the device and the single input allows considerably easier setting and dispensing of the combined compounds.

In a preferred embodiment a master drug compound, such as insulin, contained within a multiple dose, user selectable drug delivery device could be used with a single use, user replaceable, medicated module that contains a single dose of a secondary medicament and the single dispense interface. When connected to the primary drug delivery device, the secondary compound is activated/delivered on dispense of the primary compound. Although the present application specifically mentions insulin, insulin analogs or insulin derivatives, and GLP-1 or GLP-1 analogs as two possible drug combinations, other drugs or drug combinations, such as an analgesics, hormones, beta agonists or corticosteroids, or a combination of any of the above-mentioned drugs could be used with our proposed method and system.

For the purposes of our proposed method and system the term "insulin" shall mean Insulin, insulin analogs, insulin derivatives or mixtures thereof, including human insulin or a human insulin analogs or derivatives. Examples of insulin analogs are, without limitation, Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin or Des(B30) human insulin. Examples of insulin derivatives are, without limitation, B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyhepta¬decanoyl) human insulin.

As used herein the term "GLP-1" shall mean GLP-1, GLP-1 analogs, or mixtures thereof, including without limitation, exenatide (Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2), Exendin-3, Liraglutide, or AVE0010 (H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Ser-Lys-Lys-Lys-Lys-Lys-Lys-NH2).

Examples of beta agonists are, without limitation, salbutamol, levosalbutamol, terbutaline, pirbuterol, procaterol, metaproterenol, fenoterol, bitolterol mesylate, salmeterol, formoterol, bambuterol, clenbuterol, indacaterol.

Hormones are for example hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

According to an embodiment, a medicated module is attachable to a drug delivery device holding a first medicament. The medicated module includes a first needle and a retention feature fixed with the body of the medicated module. The medicated module also includes a recess in the body of the module, and a proximal end of the recess is defined by the retention feature. A piston is located in the recess and is slidably engaged with the recess. The module further includes a second medicament located in a first cavity in the recess, and this first cavity is defined by a distal end of the piston and a distal end of the recess. Further, the module includes a diverter feature and a second needle in the body of the module. The second needle is in fluid communication with the first cavity and the diverter feature.

After the medicated module is attached to the drug delivery device, (i) the first needle is in fluid communication with the drug reservoir (ii) the piston prevents fluid communication between the first needle and second needle, and (iii) the piston prevents fluid communication between the first needle and the diverter feature. During dispensing, the first medicament flows through the first needle into a second cavity in the recess, where the second cavity is defined by the retention feature and a proximal end of the piston. The first medicament forces the piston to move in a distal direction in the recess toward the distal end of the recess, and the movement of the piston forces substantially all of the second medicament to flow through the second needle. When the piston reaches a predetermined axial displacement, fluid communication between the first needle and the diverter feature is established. The first medicament may then be dispensed through the second needle. Thus, the medicated module facilitates sequential dosing of the second medicament followed by the first medicament.

According to another embodiment, a medicated module is attachable to a drug delivery device holding a first medicament. The module includes a first needle, a retention feature, and a recess within the the body of the medicated module. The recess is defined by a distal side of the retention feature, an inner wall of the medicated module, and a distal internal surface of the module. The module further includes a piston located in the recess that is slidably engaged with the inner wall of the recess. The piston has an axial hole through the piston. The module further includes a second needle fixed within the body of the medicated module. This second needle includes a closed proximal end, a first side hole, and a second side hole. The first side hole is located on a proximal side of the second needle, and the second side hole is located in close proximity to the distal internal surface. Further, the first side hole is located in the axial hole of the piston prior to dispensing. The module further includes a second medicament, wherein the second medicament is located in a first cavity in the recess, where the first cavity is defined by the distal internal surface of the medicated module and a distal end of the piston.

After the medicated module is attached to the drug delivery device, (i) the first needle is in fluid communication with the drug reservoir and (ii) the piston and the closed end of the second needle prevents fluid communication between the first and second needle. During dispensing, the first medicament flows through the first needle into a second cavity in the recess, where the second cavity is defined by the retention feature and a proximal end of the piston. The first medicament forces the piston to move in a distal direction in the recess toward the distal inner surface, and the piston forces substantially all of the second medicament to flow through the second needle. At a predetermined axial displacement of the piston, the piston travels beyond the first side hole of the second needle, thereby opening up fluid communication between the first needle and second needle. The first medicament may then be dispensed through the second needle. Thus, the medicated module facilitates sequential dosing of the medicament in the medicated module followed by the medicament in the primary device.

According to yet another embodiment, a method of dispensing a non-user settable dose of one medicament followed by a user-settable dose of a primary medicament using a single dispense interface is provided. The method includes the step of attaching a medicated module to a drug delivery device, the drug delivery device having a primary drug reservoir holding the primary medicament. The medicated module includes (i) a first needle fixed within a body of the medicated module, (ii) a retention feature fixed within the body of the medicated module, (iii) a recess within the body of the medicated module, wherein a proximal end of the recess is defined by the retention feature, (iv) a piston located in the recess, wherein the piston is slidably engaged with the recess, (v) a second medicament, wherein the second medicament is located in a first cavity in the recess, wherein the first cavity is defined by a distal end of the piston and a distal end of the recess, (vi) a diverter feature located in the body of the medicated module, and (vii) a second needle fixed within the body of the medicated module, wherein the second needle is in fluid communication with the first cavity and the diverter feature.

The method further includes the step of setting a dose of the primary medicament contained in the primary drug reservoir using a single dose setter of the drug delivery device. The method further includes the step of activating a dose button on the drug delivery device to cause the set dose of the primary medicament from the primary drug reservoir to flow in the distal direction toward the piston. During dispensing, the first medicament flows through the first needle into a second cavity in the recess. The second cavity is defined by the retention feature and a proximal end of the piston, and the first medicament forces the piston to move in a distal direction in the recess toward the distal end of the recess. The movement of the piston forces substantially all of the second medicament to flow through the second needle. When the piston reaches a predetermined axial displacement, the diverter feature transitions from a closed position to an open position, wherein fluid communication between the first needle and the diverter feature is established when the diverter feature is in the open position. Finally, the method includes the step of forcing the primary medicament to flow through the second needle. Thus, the medicated module facilitates sequential dosing of the second medicament followed by the first medicament.

A medicated module in accordance with Applicants' proposed concept can be designed for use with any drug delivery device with an appropriate compatible interface. However, it may be preferable to design the module in such a way as to limit its use to one exclusive primary drug delivery device (or family of devices) through employment of dedicated or coded features to prevent attachment of a non-appropriate medicated module to a non-matching device. In some situations it may be beneficial to ensure that the medicated module is exclusive to one drug delivery device while also permitting the attachment of a standard drug dispense interface to the device. This would allow the user to deliver a combined therapy when the module is attached, but would also allow delivery of the primary compound independently through a standard drug dispense interface in situations, such as, but not limited to, dose splitting or top-up of the primary compound.

A particular benefit of Applicants' methods and systems is that the methods and systems allow for sequential dosing of a first medicament and a second medicament through a single dispense interface. Thus, the methods and systems beneficially prevent or limit mixing of the first, primary medicament and the second medicament. This may be beneficial, for example, when mixing of medicaments negatively or detrimentally affects at least one of the medicaments.

In a preferred embodiment, the primary drug delivery device is used more than once and therefore is a multi-use device; however, the drug delivery device may also be a single use disposable device. Such a device may or may not have a replaceable reservoir of the primary drug compound, but Applicants' proposed concept is equally applicable to both scenarios. It is also possible to have a suite of different medicated modules for various conditions that could be prescribed as one-off extra medication to patients already using a standard drug delivery device. Should the patient attempt to reuse a previously used medicated module, features may be present that prevent reattachment to a primary drug delivery device or that prevent or discourage subsequent dosing through the needle via alternative means. For example, this module may include a locking needle guard that is activated after a user delivers a dose from the medicated module. Other means of alerting the user may include some (or all) of the following:
Physical prevention of medicated module re-attachment to the primary drug delivery device once the module has been used and removed.

Physical / hydraulic prevention of subsequent liquid flow through the drug dispense interface once it has been used.

Physical locking of the dose setter and/or dose button of the primary drug delivery device.

Visual warnings (e.g., change in color and/or warning text/indicia within an indication window on the module once insertion and/or fluid flow has occurred).

Tactile feedback (presence or absence of tactile features on the outer surface of the module hub following use).

A further proposed feature is that both medicaments are delivered via one injection needle and in one injection step. This offers a convenient benefit to the user in terms of reduced user steps compared to administering two separate injections. This convenience benefit may also result in improved compliance with the prescribed therapy, particularly for users who find injections unpleasant or who have computational or dexterity difficulties.

These as well as other advantages of various aspects of the present invention will become apparent to those of ordinary skill in the art by reading the following detailed description, with appropriate reference to the accompanying drawings.

### BRIEF DESCRIPTION OF THE DRAWINGS

Exemplary embodiments are described herein with reference to the drawings, in which:
Figure 1 illustrates a perspective view of one possible drug delivery device that can be used with Applicants' proposed medicated module;
Figure 2a illustrates a cross-sectional view of an exemplary medicated module attached to an exemplary drug delivery device;
Figure 2b illustrates a detailed cross-sectional view of the exemplary medicated module of Figure 2a;
Figure 3 illustrates a cross-sectional view of the exemplary medicated module of Figure 2a during dispense;
Figure 4 illustrates a cross-sectional view of the exemplary medicated module of Figure 2a during dispense;
Figure 5a illustrates a cross-sectional view of an exemplary medicated module attached to an exemplary drug delivery device;
Figure 5b illustrates a detailed cross-sectional view of the exemplary medicated module of Figure 5a; and
Figure 6 illustrates a cross-sectional view of the exemplary medicated module of Figure 5a during dispense.

### DETAILED DESCRIPTION

Applicants' proposed concept is a system and method for sequentially dispensing a non-user settable dose of one medicament followed by a user-settable dose of a primary medicament using a single dispense interface. The proposed concept relates specifically to a method and system that utilizes a piston and diverter feature that, in combination with one another, facilitate first delivering all or substantially all of a given medicament (e.g., a secondary medicament from a medicated module) through an output needle and then thereafter delivering yet another medicament (e.g., a primary medicament from a primary drug delivery device) through the same output needle in a single injection step.

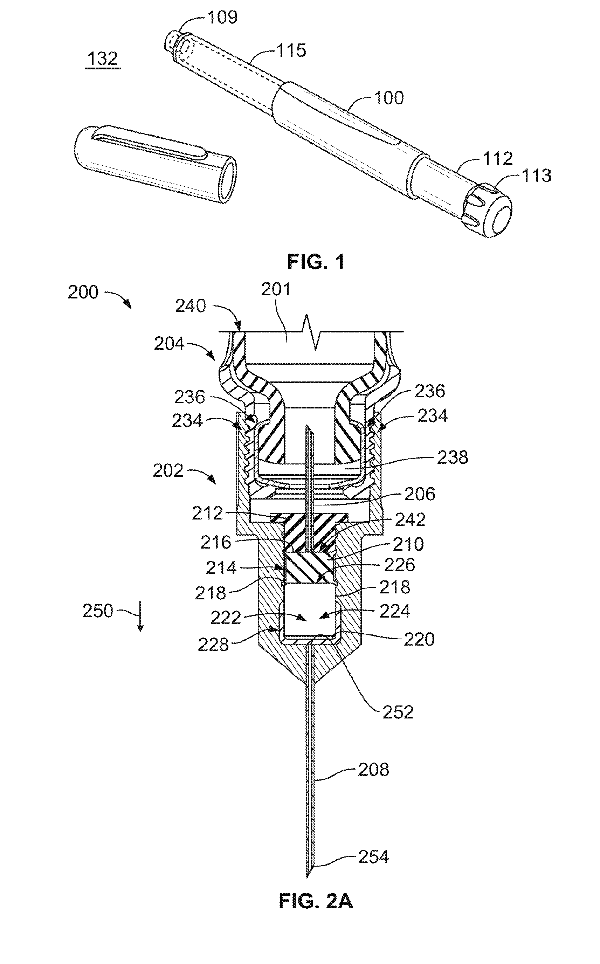

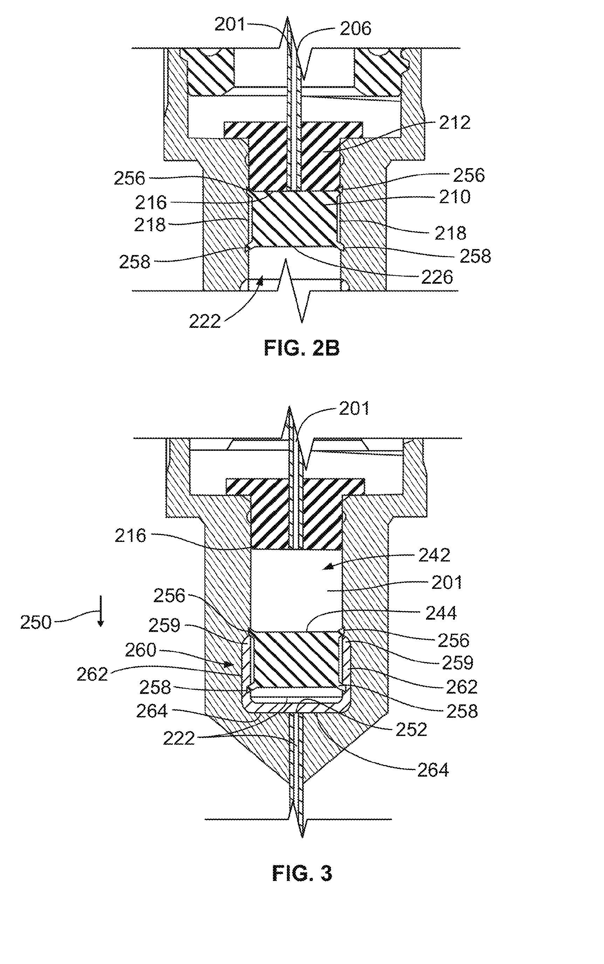

A medicated module in accordance with embodiments of Applicants' proposed concepts may be attached to a primary drug delivery device, such as drug delivery device 100. Figure 1 illustrates one example of a drug delivery device 100 to which a medicated module, such as the exemplary medicated modules depicted in Figures 2-6, can be attached. Specifically, the medicated module can be attached to the connection means 109 of distal end 132. A medicated module in accordance with Applicants' proposed concept is preferably self-contained and provided as a sealed and sterile disposable module that has an attachment means compatible to the attachment means 109 at the distal end 132 of device 100. Although not shown, the medicated module could be supplied by a manufacturer contained in a protective and sterile container, where the user would peel or rip open a seal or the container itself to gain access to the sterile medicated module. Further, the drug delivery device 100 includes a housing including a single dose setter 112. The dose setter 112 may be operably connected to a primary reservoir of medicament that may be stored in the drug delivery device, such as in cartridge holder 115. The user may use a dose dial button 113 in order to dial a user selectable dose of the primary medicament.

Applicants' proposed concept is a medicated module that is attachable to a drug delivery (such as drug delivery device 100) that has a drug reservoir holding a first, primary medicament. The proposed medicated modules include a piston and diverter feature that together facilitate sequential dosing of one medicament followed by another medicament.

A first embodiment is now described with reference to Figures 2a-b, 3, and 4. Specifically, Figure 2 illustrates a drug delivery system 200 including medicated module 202 and primary drug delivery device 204 that contains a first, primary medicament 201. In Figure 2a, only a partial view of the distal end of the primary drug delivery device 204 is shown. Primary drug delivery device 204 may be the same as or similar to drug delivery device 100 of Figure 1. The medicated module 202 includes a first needle 206 and a second needle 208. The first needle 206 may be referred to herein as an "engagement needle", as the needle engages with or communicates with the reservoir of drug delivery device 204 when the module 202 and device 204 are attached. Further, the second needle 208 may be referred to herein as an "output needle", as the second needle may be used to subcutaneously inject medicament into an injection site, such as an injection site of a user of drug delivery system 200.

The medicated module 202 also includes a piston 210 and a medicament retention feature 212. Piston 210 is located in a recess 214 within the body of the medicated module. The recess 214 is defined by the distal end 216 of the retention cap 212, inner sidewall 218 of the medicated module, and distal surface 220. Preferably, prior to dispense, the piston 210 is located just below the retention feature 212, as shown in Figures 2a-b.

Medicated module 202 also includes second medicament 222. The second medicament 222 is located in a first cavity 224 in the recess 214. The first cavity 224 is defined by a distal end 226 of the piston 210 and the distal end 220 of the recess 214. In this illustrated embodiment, there is no difference between the distal end of the recess and the distal end of the radial portion of the diverting channel other than a larger diameter. It is this locally larger diameter (at a defined axial position in the recess 214) that causes a gap/channel between the piston 210 and inner recess wall. It is this increase in diameter that allows the primary medicament 201 to flow around the piston 210. The diameter could be larger all the way around the piston 210 but preferably may be larger at only a small portion or sector (similar to a bypass channel). This local small channel would help minimize the ullage of primary medicament 201 in the system after dispense. Alternatively, this local small channel may comprise a plurality of local vertical channels/groves. In such an arrangement, an optimum number could be based upon making sure that the dispense force was not too high and that the ullage was as low as possible. This channel configuration could be separated by a surface. It should be noted that there could be channels running from the inner recess wall of the recess/diverter channels to the central output needle 208. Such a channel configuration could prevent the piston 210 from blocking off the output needle 208. As such, the piston 210 is sealed to a cylindrical surface until it reaches a defined axial displacement. At this point, the sealing surface steps away either locally or as a complete stepped diameter. This step or local recess breaks the seal such that fluid can flow around the piston 210 to reach the outlet needle 208.

As will be described in greater detail below, cavity 224 is a dynamically-sized cavity, such that the size will change during the dispensing process due to axial movement of the piston 210.

The medicated module 202 also includes a diverter feature 228 located in the body of the medicated module. As will be described in more detail below, the diverter feature and the piston 210 in combination facilitate sequential dosing of the second medicament 222 followed by the first medicament 201. The diverter feature 228 comprises a diverting channel. Generally, the diverter feature 228 serves as a channel for the first medicament 201 after the secondary medicament is substantially fully dispensed from the module 202. This diverter is described in more detail below in reference to the dispensing process.

The medicated module 202 also includes an attachment means 234. The attachment means 234 is configured to attach to a corresponding attachment means of a drug delivery device, such as the attachment means 236 of drug delivery device 204 (or, in another example, the attachment means 109 at the distal end 132 of device 100). Further, in a preferred embodiment, the medicated module 202 also includes a needle cover or needle guard (not shown). A needle cover may have a connection feature (e.g., a snap-fit feature) that allows the cover to be removably attached to the body of the medicated module 202. A needle cover or needle guard may substantially conceal the second needle 208 from a user's view so as to beneficially reduce any needle anxiety that a patient may be experiencing. While substantially concealing the needle, the needle cover or needle guard also helps to prevent inadvertent needle sticks.

Figures 2a and 2b depict the medicated module 202 after the module 202 is attached to drug delivery device 204 and prior to the dispense process. Attachment of medicated module 202 to drug delivery device 204 causes the engagement needle 206 to penetrate the septum 238 of the drug cartridge or reservoir 240 of the drug delivery device 204. Once the engagement needle 206 has passed through the septum 238 of the cartridge 240, fluid connection is made with the first, primary medicament 201. In other words, the first needle 206 is in fluid communication with the drug reservoir 240. At this stage, the piston 210 separates the first needle 206 and second needle 208, thereby preventing fluid communication between the two needles 206, 208. Thus, the first medicament 201 does not interact with (e.g., mix with) second medicament 222.

After the module 202 is attached to the device 204, a user may set a user-settable dose of the first medicament 201. The dose of the drug delivery device may be set in a usual manner (e.g., by dialing out an appropriate number of units of the primary medicament 201 with a dose dial). Dispense of the second medicament 222 followed by the first, primary medicament 201 may then be achieved via activation of the dosing mechanism of the drug delivery device. The sequential dispense of the medicaments 222, 201 is described with reference to Figures 2a, 2b, and 3-4.

As the primary medicament 201 is dispensed from the reservoir 240, the medicament 201 flows distally in direction 250 through the engagement needle 206 into a second cavity 242. Cavity 242 is a cavity formed between the proximal end 244 of the piston 210 and the distal end 216 of the retention feature 212. Retention feature 212 may also be referred to herein as a "retention cap", as the retention feature 212 forms a "cap" over the piston 210, retaining the medicament 201 in the cavity 242.

The retention cap 212 is preferably substantially rigid. As an example, the retention cap may be composed of substantially rigid materials that are compatible with the drug formulation that comes into contact with the retention cap. The retention cap could therefore be any number of rigid materials depending upon the medicament in use. It is foreseeable that this cap could be an engineering polymer (e.g. Polypropylene - PP, Acrylonitrile Butadiene Styrene - ABS, Cyclo Olefin Polymer - COP, Polyethylene terephthalate - PET, Polycarbonate - PC, Polyoxymethylene - POM, Low Density Polyethylene - LDPE and others) or even Stainless steel. Since the retention cap 212 is substantially rigid and the first medicament 201 is generally or effectively incompressible, the piston 210 is displaced axially in distal direction 250 as the first medicament 201 is dispensed into cavity 242. As mentioned above, the retention cap 212 is fixed in the medicated module 202. Thus, the retention cap 212 will remain stationary when the first medicament 201 is forced into the dynamically-sized cavity 242.

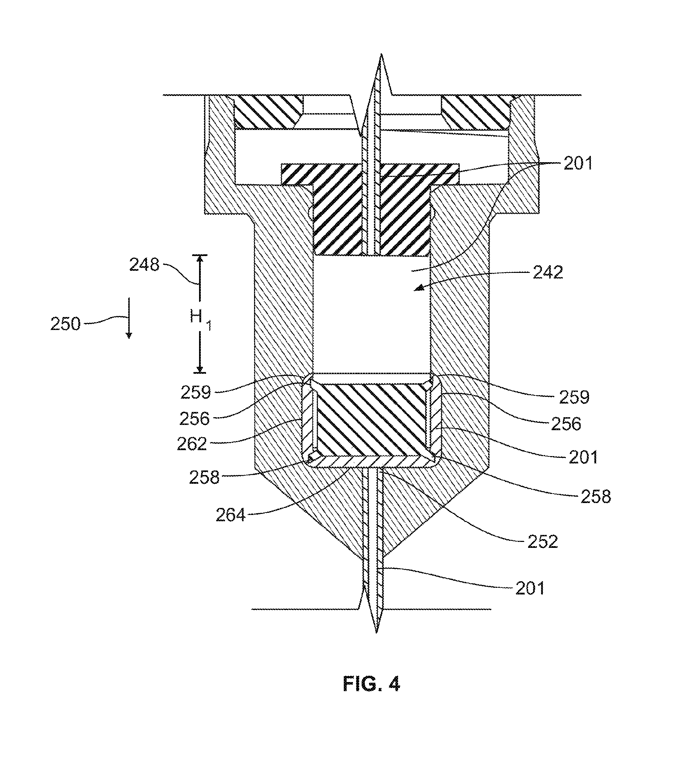

As mentioned above, the size of the cavity 242 is dynamic. That is, the cavity 242 changes size (i.e., height) throughout the dosing process. Specifically, the cavity 242 changes from a height of approximately zero (as shown in Figures 2a and 2b) to a height of H1 248 (as shown in Figure 4). The height 248 corresponds to the amount of distal travel of the piston 210 during the dosing process. Additionally, the size of the first cavity 224 is similarly dynamic (i.e., the height of cavity 224 decreases as the height of cavity 242 increases).

In the example of Figures 2a-b and 3-4, the piston 210 is preferably a "floating piston". That is, the piston is not connected to a piston rod and is free to move axially in the body of the medicated module upon an axially-directed force. It should be understood that although the piston 210 is referred to as a floating piston, the piston 210 is securely held in the body. Specifically, the floating piston is in sliding and fluid-tight engagement or substantially fluid-tight engagement with the medicated module. Specifically, the first portion 212 is in sliding and substantially fluid-tight engagement with the sidewall 218 of the recess 214. Therefore, the piston 210 prevents (i) the primary medicament 201 from flowing in the distal direction beyond the piston and (ii) the secondary medicament 222 from flowing in the proximal direction beyond the piston. The fluid-tight engagement may be accomplished with top sealing feature 256 and bottom sealing feature 258. These sealing features 256, 258 provide sliding fluid-tight engagement with the sidewall 218 of recess 214.

The fluid-tight engagement, however, still allows for the piston 210 to slide axially upon the force of primary medicament 201 flowing into cavity 242 when the medicament is dispensed by a user. As seen when Figures 2a and 2b are compared to Figure 3, dispense of the first medicament 201 forces the piston 210 to move in the distal direction 250. This distal movement forces the second medicament 222 to flow to and thereafter out of second needle 208. Specifically, the movement of the piston 210 causes the second medicament 222 to flow through opening 252 of the output needle. The medicament 222 is then forced through the needle 208 and out of output 254 at the distal end of the needle 208 into an injection site. The distal end 220 of the recess 214 includes a hole so that medicament may flow from the recess 214 to the opening 252 of the output needle 208.

When the piston 210 reaches a predetermined axial displacement, fluid communication between the first needle 206 and the diverter 228 is established. This predetermined axial displacement preferably corresponds to the second medicament 222 being substantially fully dispensed. It should be understood that although preferably substantially all of the second medicament 222 is dispensed before the fluid communication between needle 206 and diverter 228 (and therefore between needle 206 and needle 208) is opened up, it is not necessary that 100% of the second medicament is dispensed from the medicated module. For example, in an embodiment, 90-95% of medicament 222 may be dispensed from the medicated module 202 before the fluid communication between needle 206 and diverter 228 is opened up. Other examples are possible as well. Further, it should be understood that some of the second medicament 222 may still be in needle 208 when the fluid communication between needle 206 and diverter 228 is opened up. Still further, a small amount of the second medicament 222 may still be in the cavity 224 when the piston reaches the predetermined axial displacement.

In order to establish fluid communication between the first needle 206 and the diverter 228, sealing features 256 of the floating piston axially traverse past an opening 259 of the diverter feature 228. Opening 259 is shown in Figures 3 and 4. In this example, the diverter feature 228 comprises a diverting channel 260. The opening 259 of the diverting channel 260 may be a hole/groove or collection of circumferential holes/grooves in the inner wall 218. The diverting channel 260 is located around a distal portion of the recess 214. As shown in Figures 2a and 2b, prior to injection the diverting channel 260 is located around first cavity 224. Diverting channel 260 includes an axial channel portion 262 and a radial channel portion 264. The axial channel portion is located around the inner wall 218 of recess 214, and the radial channel portion 264 is located below the distal internal surface 220. In particular, the axis of the channel 260 is parallel to the axis of floating piston 210 and extends vertically downwards to the distal surface 220, whereby the channel 260 extends radially inwards to connect with the output needle 208.

As shown in Figure 3, when top sealing features 256 are located just above the opening 259 of the diverting channel 260, the piston 210 prevents fluid communication between the engagement needle 206 and the output needle 208. However, when the piston 210 is forced further axially, as shown in Figure 4, fluid communication is established between the two needles via the diverting channel 260. In particular, the first medicament 201 flows through opening 259 into diverting channel 260, through axial portion 262 and radial portion 264 to the opening 252 of the output needle 208. The medicament 201 may then be forced out of the output needle 208 into an injection site.

It should be noted that the flow of first medicament 201 through the diverting channel 260 may force out any second medicament 222 that may have been present in the diverting channel 260.

After the user finishes dispensing the first medicament 201, the user may remove the output needle 208 from the injection site. Then, the depleted medicated module 202 may be disposed of. Assuming that the drug delivery device 204 still holds some first medicament 201, the drug delivery device 204 may be reused by the patient as required.

An alternative embodiment of Applicants' proposed floating piston and diverter concept includes a diverting channel that includes an annulus between the floating piston and the internal surface of a medicated module for the entire perimeter of the sealing feature. For example, if the cross-section of the piston is circular, the internal diameter of the medicated module will be greater than the diameter of the sealing features located on the piston. The region where the annulus of fluid is present will be limited to the diverting portion. Prior to this region, the sealing features of the floating piston provide a fluid seal to the internal wall of the medicated module.

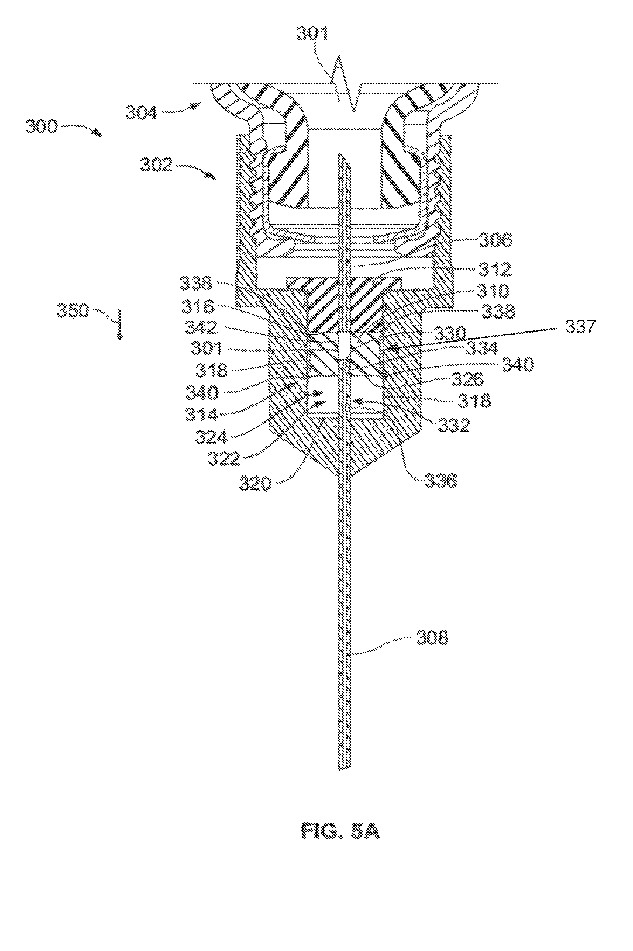

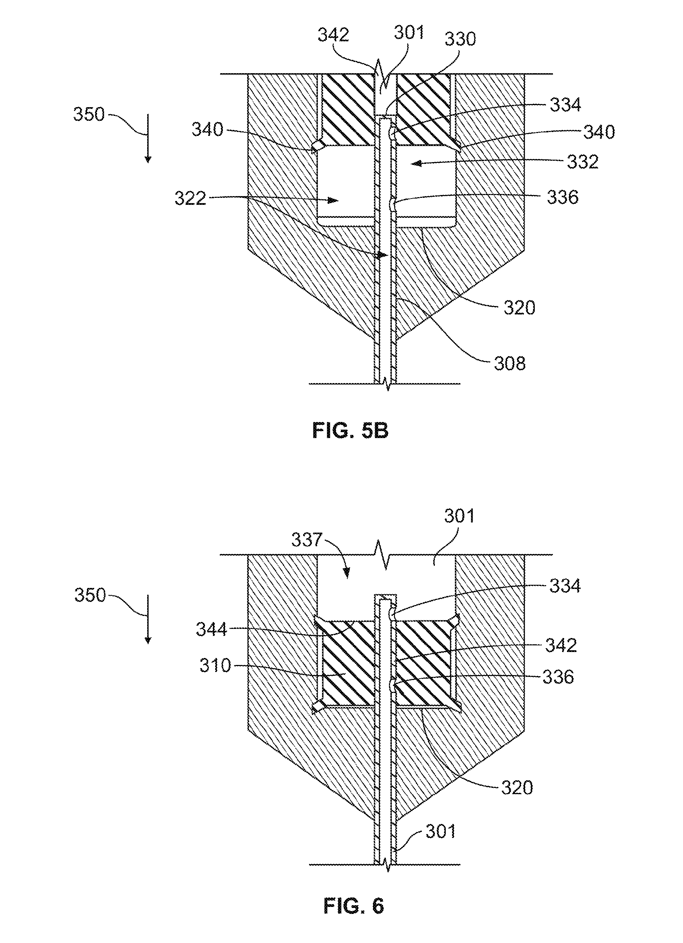

Figures 5a, 5b, and 6 depict an alternative embodiment of Applicants' proposed floating piston and diverter concept. Similar to the embodiments discussed above, this alternative embodiment includes a piston and a diverting channel that facilitates sequential delivery of a secondary medicament from a medicated module followed by a first primary medicament from a drug delivery device. However, in this embodiment, the diverting channel is formed by the output needle itself in lieu of a flow path (i.e., diverting channel) between the piston and the medicated module. Specifically, the output needle is configured in a way such that its interaction with the floating piston allows the primary medicament to only flow through the output needle after all or substantially all of the secondary medicament is delivered.

The later embodiment is now described with reference to Figures 5a, 5b, and 6. Specifically, Figure 5 illustrates a drug delivery system 300 including medicated module 302 and primary drug delivery device 304 that contains a first, primary medicament 301. This drug delivery system 300 is similar in many respects to drug delivery system 200, and thus is not described in as great of detail. It should be explicitly noted, however, that many of the possibilities and permutations discussed above with reference to system 200 may equally apply to system 300. Similar to system 200, system 300 facilitates delivery of a secondary medicament followed by delivery of a first primary medicament. However, the diverter feature in system 300 is different than the diverter feature in system 200.

The medicated module 302 includes engagement needle 306 and output needle 308. The medicated module 302 also includes floating piston 310 and medicament retention feature or retention cap 312. The piston 310 is located in recess 314 within the body of the medicated module. The recess is defined by the distal end 316 of the retention cap 312, sidewall 318 of the medicated module, and distal internal surface 320. Medicated module 302 also includes second medicament 322, located in first cavity 324 of recess 314. The first cavity is defined by the distal end 326 of piston 310 and the distal surface 320. Similar to first cavity 224, cavity 324 is a dynamically-sized cavity that depends on the location of piston 310 in the recess 314 during the dispense process.

The output needle is configured such that the output needle will function as a diverting channel to facilitate sequential dosing of second medicament 322 followed by primary medicament 301. As shown in Figures 5a and 5b, the output needle 308 protrudes from the distal surface 320 of the medicated module 302. This protruding end is generally referred to as protruding end 332. So that the needle 308 is able to function as a diverter, the end 330 of the protruding end 332 of the needle 308 is closed. Further, the protruding end 332 includes two side holes - top side hole 334 and bottom side hole 336. The axis of each side hole is preferably perpendicular the output needle's axis. The bottom hole 336 is located near the distal surface 320 of the medicated module 302, and the top hole 334 is located near the closed end 330 of the protruding end 332. In other words, the top side hole is located on a proximal end of the needle 308 in close proximity to end 330, and second side hole is located in close proximity to the distal internal surface 320. For example, first side hole may be located within 1 - 2 millimeters of the closed end 330, and second side hole may be located within 1- 2 millimeters of the distal internal surface 320. Ideally each hole may be located as close to end face as possible. This hole location would help minimize ullage of the secondary medicament 322. The distance between the holes is one feature that helps to ensure functionality, sequential dispense and minimize mixing. For example, the distance between the first and second hole, 334 and 336, maybe a function of the thickness of bung, volume of secondary medicament 322 at start, and ullage at end with allowance for tolerances. In one arrangement, the distance between the first and the second holes, 334 and 336, should be such that as the piston 310 starts to close off the distal hole 336, the proximal hole 334 starts to be opened. Such a preferred sequence allows the second medicament 322 to flow until the piston moves sufficiently axially enough to have dispensed substantially all of the second medicament 322 at which point it starts to block the route for the second medicament and open up the hole for primary medicament to enter.

During dispense, the primary medicament 301 is dispensed into the cavity 337 formed between the retention cap 312 and the proximal end of floating piston 310. As the needle end 330 is closed and a fluid seal is achieved by the sealing features 338 and 340 of the floating piston 310, the piston 310 is displaced axially in direction 350. This displacement causes the secondary medicament 322 to flow through the lower side hole 336 into the needle 308, through the needle 308, and into an injection site. As the piston 310 is displaced axially, the needle 308 traverses through a hole 342 arranged in the floating piston 310. In this example, the hole 342 is located in the center of the piston 310. Further, in this example, the hole 342 extends through the entire piston 310. Thus, after the module 302 is connected to device 304, some primary medicament 301 will enter this hole 342. However, due to the closed end 330 and the needle 308 being in substantially fluid-tight engagement with the piston 310, the primary medicament 301 will not interact with the secondary medicament 322, as shown in Figure 5b.

At a predetermined axial displacement, the top side hole 334 clears the top surface 344 of the floating piston 310, as illustrated in Figure 6. This predetermined axial displacement preferably corresponds to the second medicament 322 being substantially fully dispensed from the module. When top side hole 334 clears the top surface 344, fluid communication is opened up between the engagement needle 306 and the output needle 308. Primary medicament 301 may then flow through top side hole 334 into the output needle 308, through the output needle 308, and into an injection site. As shown in Figure 6, when top side hole 334 is clear of the top surface 344, the bottom side hole 336 is located in the hole 342 of the piston 310. Such location essentially plugs the bottom side hole 336. Thus, primary medicament will not flow out the side hole 336 as the medicament 301 passes by side hole 336. Therefore, beneficially, the closed end 330 of the needle 308 and the upper side hole 334 (in combination with floating piston 310) allow the output needle 308 to function as a diverting channel that facilitates sequential dosing of two medicaments.

Applicants' proposed concept also includes a method for dispensing a non-user settable dose of one medicament followed by a user-settable dose of a primary medicament. The method includes the step of attaching a medicated module to a drug delivery device, such as the medicated modules and drug delivery devices illustrated in Figures 1-6. As described above, the drug delivery device has a primary drug reservoir holding the primary medicament. Further, the medicated module includes a first needle, a second needle, a floating piston, a second medicament, and a diverter feature.

The method includes the step of attaching the medicated module to a drug delivery device, the drug delivery device having a primary drug reservoir holding the primary medicament. Specifically, the medicated module includes (i) a first needle fixed within a body of the medicated module, (ii) a retention feature fixed within the body of the medicated module, (iii) a recess within the body of the medicated module, wherein a proximal end of the recess is defined by the retention feature, (iv) a piston located in the recess, wherein the piston is slidably engaged with the recess, (v) a second medicament, wherein the second medicament is located in a first cavity in the recess, wherein the first cavity is defined by a distal end of the piston and a distal end of the recess, (vi) a diverter feature located in the body of the medicated module, and (vii) a second needle fixed within the body of the medicated module, wherein the second needle is in fluid communication with the first cavity and the diverter feature.

The method further includes the step of setting a dose of the primary medicament contained in the primary drug reservoir using a single dose setter of the drug delivery device. The method further includes the step of activating a dose button on the drug delivery device to cause the set dose of the primary medicament from the primary drug reservoir to flow in the distal direction toward the piston, wherein, during dispensing, the first medicament flows through the first needle into a second cavity in the recess. The second cavity is defined by the retention feature and a proximal end of the piston, and the first medicament forces the piston to move in a distal direction in the recess toward the distal end of the recess. The movement of the piston forces substantially all of the second medicament to flow through the second needle, and wherein, when the piston reaches a predetermined axial displacement, the diverter feature transitions from a closed position to an open position, wherein fluid communication between the first needle and the diverter feature is established when the diverter feature is in the open position. Finally, the method includes the step of forcing the primary medicament to flow through the second needle.

For instance, the diverter feature may be the diverting channel discussed in reference to Figures 2-4. The closed position may be when the piston prevents fluid communication between the first needle and the diverting channel, and the open position may be after fluid communication is opened up when the piston traverses past an opening to the diverting channel. In another example, the diverter feature may be the diverting channel discussed in reference to Figures 5-6. The closed position may be when the top side hole of the output needle is located in the axial hole of the piston, and the open position may be after fluid communication is opened up between the first and second needle when the top side hole traverse past the top of the piston.

As described above, Applicants' proposed concept beneficially allows for sequential dosing of a secondary medicament stored in a medicated module followed by a primary medicament from a primary drug delivery device. The proposed diverting features help to ensure that the primary medicament is only delivered after the secondary medicament is substantially dispensed. Thus, beneficially, the proposed concept prevents or limits the mixing of the two medicaments within the device and during delivery. Avoidance of mixing of the 2 drug formulations during delivery might have several advantages. For example, it is known that the pharmacokinetics of certain drugs is critically dependent on their concentration. By delivering 2 drugs sequentially with no mixing the optimal concentration of each drug for optimal pharmacokinetics can be maintained. In addition, certain drugs have to be formulated in particular solvent environments (e.g., a specific pH range) to remain in solution. By delivering 2 drugs sequentially with no mixing the optimal pH range and therefore solubility can be maintained during delivery.

Further, Applicants' proposed concept has the additional benefit of sequentially dosing the medicaments so that the drug concentration within the ullage in the needle after use is consistent.

The connection or attachment between the medicated module of the above described embodiments may contain additional features (not shown), such as connectors, stops, splines, ribs, grooves, and the like design features, that ensure that specific medicated module are attachable only to matching drug delivery devices. Such additional features would prevent the insertion of a non-appropriate medicated module to a non-matching injection device.

The shape of the medicated module may be a cylindrical body or any other geometric shape suitable for defining a fluid reservoir or for containing discrete self-contained reservoir of the medicament in the medicated module and for attaching one or more needle cannula. The medicated module can be manufactured from glass or other drug contact suitable material. The integrated output needle can be any needle cannula suitable for subcutaneous or intramuscular injection. Preferably the medicated module is provided by a drug manufacturer as a stand-alone and separate device that is sealed to preserve sterility. The sterile seal of the module is preferably designed to be opened automatically, e.g. by cutting, tearing or peeling, when the medicated module is advanced or attached to the drug delivery device by the user.

The medicated module of Applicants' concept should be designed to operate in conjunction with a multiple use injection device, preferably a pen-type multi-dose injection device, similar to what is illustrated in Figure 1. The injection device could be a reusable or disposable device. By disposable device it is meant an injection device that is obtained from the manufacturer preloaded with medicament and cannot be reloaded with new medicament after the initial medicament is exhausted. The device may be a fixed dose or a settable dose and preferably a multi-dose device, however, in some cases it may be beneficial to use a single dose, disposable device.

A typical drug delivery device contains a cartridge or other reservoir of medication. This cartridge is typically cylindrical in shape and is usually manufactured in glass. The cartridge is sealed at one end with a rubber bung and at the other end by a rubber septum. The drug delivery pen is designed to deliver multiple injections. The delivery mechanism is typically powered by a manual action of the user, however, the injection mechanism may also be powered by other means such as a spring, compressed gas or electrical energy.

Exemplary embodiments of the present invention have been described. Those skilled in the art will understand, however, that changes and modifications may be made to these embodiments without departing from the true scope and spirit of the present invention, which is defined by the claims.

## Claims

1. A medicated module (202, 302) attachable to a drug delivery device, the drug delivery device having a drug reservoir holding a first medicament, the medicated module (202, 302) comprising:
a first needle (206, 306) fixed within a body of the medicated module (202, 302);
a retention feature (212, 312) fixed within the body of the medicated module (202, 302);
a recess (214, 314) within the body of the medicated module (202, 302), wherein a proximal end of the recess (214, 314) is defined by the retention feature (212, 312);
a piston 210, 310) located in the recess (214, 314), wherein the piston (210, 310) is slidably engaged with the recess (214, 314);
a second medicament (222, 322), wherein the second medicament (222, 322) is located in a first cavity (224, 324) in the recess (214, 314), wherein the first cavity (224, 324) is defined by a distal end (226, 326) of the piston (210, 310) and a distal end (220, 320) of the recess (214, 314); and
a diverter feature (228) located in the body of the medicated module (202, 302);
and
a second needle (208, 308) fixed within the body of the medicated module (202, 302), wherein the second needle (208, 308) is in fluid communication with the first cavity (224, 324) and the diverter feature (228),
wherein, after the medicated module (202, 302) is attached to the drug delivery device, (i) the first needle (206, 306) is in fluid communication with the drug reservoir (ii) the piston (210, 310) prevents fluid communication between the first needle (206, 306) and second needle (208, 308), and (iii) the piston (210, 310) prevents fluid communication between the first needle (206, 306) and the diverter feature (228),
wherein, during dispensing, the first medicament flows through the first needle (206, 306) into a second cavity (242, 337) in the recess (214, 314), wherein the second cavity (242, 337) is defined by the retention feature (212, 312) and a proximal end of the piston (210, 310), and wherein the first medicament forces the piston (210, 310) to move in a distal direction toward the distal end (220, 320) of the recess (214, 314), wherein the movement of the piston (210, 310) forces substantially all of the second medicament (222, 322) to flow through the second needle (208, 308), and
wherein, when the piston (210, 310) reaches a predetermined axial displacement, fluid communication between the first needle (206, 306) and the diverter feature (228) is established.

2. The medicated module (202, 302) of claim 1, wherein after the fluid communication is established, the first medicament is dispensed through the second needle (208, 308).

3. The medicated module (202, 302) of claim 1 or claim 2, wherein fluid communication between the first needle (206, 306) and the diverter feature (228) is established when the piston (210, 310) traverses past an opening (259) of the diverter feature (228).

4. The medicated module (202, 302) of any of the preceding claims, wherein, when the piston (210, 310) reaches the predetermined axial displacement, substantially all of the second medicament (222, 322) is dispensed from the first cavity (224, 324).

5. The medicated module (202, 302) of any preceding claims wherein the diverter feature (228) comprises an axial channel portion (262) and a radial channel portion (264), wherein the axial channel portion (262) is located around a portion of an inner wall (218, 318) of the recess (214, 314), and the radial channel portion (264) is located below the distal end (220, 320) of the recess (214, 314).

6. The medicated module (202, 302) of claim 5, wherein an opening (252) of the second needle (208, 308) is connected to the radial channel portion (264).

7. The medicated module (202, 302) of claim 3, wherein the opening (259) of the diverter feature (228) is a hole in an inner wall (218, 318) of the recess (214, 314).

8. The medicated module (202, 302) of any of the preceding claims, wherein the piston (210, 310) comprises at least one sealing feature (256, 258; 338, 340).

9. A medicated module (202, 302) attachable to a drug delivery device, the drug delivery device having a drug reservoir holding a first medicament, the medicated module (202, 302) comprising:
a first needle (206, 306) fixed within a body of the medicated module (202, 302);
a retention feature (212, 312) fixed within the body of the medicated module (202, 302);
a recess (214, 314) within the body, wherein the recess (214, 314) is defined by a distal end (216, 316) of the retention feature (212, 312), an inner wall (218, 318) of the medicated module (202, 302), and a distal internal surface (220, 320) of the module (202, 302);
a piston (210, 310) located in the recess (214, 314), wherein the piston (210, 310) slidably engaged with the inner wall (218, 318) of the recess (214, 314), and wherein the piston (210, 310) has an axial hole (342);
a second needle (208, 308) fixed within the body of the medicated module (202, 302), wherein the second needle (208, 308) comprises a closed proximal end (330), a first side hole (334), and a second side hole (336), wherein the first side hole (334) is located on a proximal end of the second needle (208, 308), wherein the second side hole (336) is located in close proximity to the distal internal surface (220, 320), and wherein the first side hole (334) is located in the axial hole (342) prior to dispensing;
a second medicament (222, 322), wherein the second medicament (222, 322) is located in a first cavity (224, 324) in the recess (214, 314), wherein the first cavity (224, 324) is defined by the distal internal surface (220, 320) of the medicated module (202, 302) and a distal end (226, 326) of the piston (210, 310),
wherein, after the medicated module (202, 302) is attached to the drug delivery device, (i) the first needle (206, 306) is in fluid communication with the drug reservoir and (ii) the piston (210, 310) and the closed proximal end (330) of the second needle (208, 308) prevent fluid communication between the first and the second needle (206, 306; 208, 308),
wherein, during dispensing, the first medicament flows through the first needle (206, 306) into a second cavity (242, 337) in the recess (214, 314), wherein the second cavity (242, 337) is defined by the retention feature (212, 312) and a proximal end of the piston (210, 310), and wherein the first medicament forces the piston (210, 310) to move in a distal direction toward the distal internal surface (220, 320), and wherein the piston (210, 310) forces substantially all of the second medicament (222, 322) to flow through the second needle (208, 308), and
wherein, at a predetermined axial displacement of the piston (210, 310) the piston (210, 310) travels beyond the first side hole (334) of the second needle (208, 308), thereby opening up fluid communication between the first and the second needle (206, 306; 208, 308).

10. The medicated module (202, 302) of claim 9, wherein, when the piston (210, 310) reaches the predetermined axial displacement of the piston (210, 310), substantially all of the second medicament (222, 322) is dispensed from the first cavity (224, 324).

11. The medicated module (202, 302) of claim 9 or claim 10, wherein the piston (202, 302) is a floating piston.

12. The medicated module (202, 302) of any of claims 9-11, wherein the second needle (208, 308) is in sliding fluid-tight engagement with the piston (210, 310) when the second needle (208, 308) is in the axial hole (342).

13. The medicated module (202, 302) of claim 12, wherein, after the piston (210, 310) travels beyond the first side hole (334) of the second needle (208, 308), the second side hole (336) of the second needle (208, 308) is located in the axial hole (342).

14. The medicated module (202, 302) of claim 13, wherein the fluid-tight engagement prevents the first medicament from flowing out the second side hole (336).
